# EUROPEAN PATENT APPLICATION

(11) **EP 2 426 613 A1**
(43) Date of publication of application: **07.03.2012**
(21) Application number: 10168067.6
(22) Date of filing: 01.07.2010
(51) Int. Cl.: G06F 19/00

(54) **Metabolic analysis apparatus and metabolic analysis method**

(30) Priority: 31.07.2009 JP 2009179955
(71) Applicant: Fujitsu Limited, Kawasaki-shi, Kanagawa 211-8588 (JP); Tohoku University, Aoba-ku Sendai-shi Miyagi 980-8577 (JP)
(72) Inventor: Ciloy, Jose Martin, Fukuoka-shi, Fukuoka 814-8589 (JP); Kitajima, Masato, Fukuoka-shi, Fukuoka 814-8589 (JP); Yamazoe, Yasushi, Sendai-shi, Miyagi 980-8577 (JP)
(74) Representative: Stebbing, Timothy Charles

(57) **Abstract**

A ring structure of a predicted target compound which is in a two-dimensional structural formula is arranged so as to be in an identical position as a ring structure of a template produced by superimposing two-dimensional structural formulae of compounds capable of forming a complex with a predetermined enzyme. Among predicted target compounds overlapping with a metabolic target site of the template, predicted target compounds other than those determined as being unable to access a metabolic active center site of the enzyme, or those having the same charge as that of the metabolic active center site, are determined to be metabolizable compounds. Based on the level of contribution of each atom included in the template during a metabolic reaction of the enzyme, a structural formula of a compound obtained after the metabolic reaction of the metabolizable compound whose metabolic target site has been determined is determined.

## Description

The present invention relates to a metabolic analysis apparatus and a metabolic analysis method.

It has long been known that compounds absorbed in the body are discharged out of the body after metabolic reactions by enzyme groups relating to xenobiotic metabolism, or are changed into metabolically active compounds (reactive metabolites). Therefore, in drug discovery research and safety evaluation of chemical substances, it is important not only to search for a novel compound having a pharmacological activity, but also to predict whether the novel compound can be metabolized by an enzyme group relating to xenobiotic metabolism. In addition, if the novel compound is metabolizable, it is important to predict what kind of metabolites will be produced.

For example, if a novel compound is not metabolized by an enzyme group relating to xenobiotic metabolism, the novel compound can remain in the body, thereby causing an adverse effect on the body. Therefore, it is necessary to modify the novel compound so that it can be metabolized.

Furthermore, even in the case where a novel compound is metabolized by an enzyme group relating to xenobiotic metabolism, if there is a possibility that a metabolite can have an adverse effect on the body, it is necessary to modify the novel compound so that the metabolite does not have an adverse effect on the body. Moreover, if the pharmacological activity of the novel compound is lost in the metabolite, it is necessary to modify the novel compound so as to improve the sustainability of the pharmacological effect.

Specifically, predicting whether metabolism will occur by an enzyme group relating to xenobiotic metabolism, and the resultant metabolites, are important processes in order to improve the efficiency of drug discovery research.

A known example of such an enzyme group relating to xenobiotic metabolism is cytochrome P450 (hereinafter, referred to as "P450"). P450 is an enzyme group which plays a role in activating oxygen and metabolizing a compound into a highly water-soluble metabolite, thereby discharging the compound from the body by excretion.

For example, P450 includes enzymes metabolizing ethanol, and enzymes metabolizing the caffeine in coffee. In addition, about a hundred kinds of P450 enzymes which metabolize medicines are known. For example, even P450 enzymes capable of metabolizing several dozen kinds of medicine are known.

Accordingly, regarding P450, a method is known for predicting a metabolic reaction for an unknown compound based on known metabolic reaction data. Further, a method is also known for predicting a metabolic reaction for an unknown compound based on a docking simulation between a compound and the steric structure of P450 (regarding these methods, see, for example, Atsushi Tomonaga, "P450, Drug Metabolism Database, & Metabolite Prediction", Special Edition "Life Science", Fujitsu, 53, 5: 402-406, (09, 2002)). For example, in the method for predicting a metabolic reaction based on known metabolic reaction data, changes in structure before and after metabolic reaction for a known reaction substrate are determined in advance. If there is a pre-reaction structure of the known reaction substrate in the compound of the predicted target, that compound is determined as being metabolizable by P450. Therefore, the metabolites are predicted by applying the post-reaction structure of the known reaction substrate to the compound of the predicted target.

Further, in the method for predicting a metabolic reaction based on docking simulation, prediction of whether metabolism will occur by P450 and prediction of the metabolites produced by P450 are carried out by simulating whether the steric structure of the compound can access an active center site of the P450 steric structure.

However, in the above-described method based on known metabolic reaction data, even when the compound of the predicted target is metabolizable by P450, if there is no corresponding structure in the known reaction substrate, the compound will be predicted as being non-metabolizable. In addition, in the above-described method based on known metabolic reaction data, since the structure of a compound predicted as being metabolizable is replaced with the post-reaction structure of a known reaction substrate to predict metabolites, false positives can occur in which the predicted metabolite is not produced in the actual reaction.

In the above-described method for predicting a metabolic reaction based on a docking simulation, as the known steric structure of P450 is immobilized for use, predictions capable of handling the flexibility of structural changes in the biological environment of proteins are impossible. Furthermore, for P450 having an unknown steric structure, the metabolic reaction has to be predicted by first predicting the steric structure based on similarity to the primary sequence of the amino acid.

Specifically, the above-described conventional methods suffer from the problem that a metabolic reaction cannot be easily predicted.

Further, not only P450, but other enzymes relating to xenobiotic metabolism, as described above, also suffer from the problem that a metabolic reaction cannot be easily predicted. Moreover, not only drug discovery research, but ordinary research and development also suffers from the same problem even if a metabolic reaction for a metabolic enzyme is predicted.

Accordingly, it is desirable to provide a metabolic analysis program, a metabolic analysis apparatus, and a metabolic analysis method which enable a metabolic reaction to be easily predicted.

According to an aspect of the invention, a metabolic analysis apparatus includes an arrangement processing unit that arranges a predetermined structure of a predicted target compound which is in a two-dimensional structural formula so as to be in an identical position with a predetermined structure of a template produced by superimposing two-dimensional structural formulae of a plurality of compounds capable of forming a complex with a predetermined enzyme; and a determination unit that determines, if the two-dimensional structural formula of the predicted target compound arranged on the template by the arrangement processing unit overlaps with a metabolic target site of the predetermined enzyme in the template, that the predicted target compound is a metabolizable compound which can be metabolized by the predetermined enzyme.

Another aspect of the present invention provides a metabolic analysis program as defined in the accompanying claims. Such a program may be stored on a computer-readable medium.

Reference is made, by way of example only, to the accompanying drawings in which:
FIG. 1 is a block diagram illustrating a configuration of a metabolic analysis apparatus according to a first embodiment;
FIG. 2 depicts examples of a compound used in template production;
FIG. 3 is a diagram illustrating an example of template production;
FIG. 4 is a diagram illustrating an example of an arrangement processing unit according to the first embodiment;
FIG. 5 is a diagram illustrating a metabolizable compound;
FIG. 6 is a diagram illustrating a constraint;
FIG. 7 is a diagram illustrating a score calculation unit;
FIG. 8 is a diagram illustrating a score calculation example and a metabolite prediction example according to the first embodiment;
FIG. 9 is a diagram illustrating a sequence of metabolite determination processing performed by the metabolic analysis apparatus according to the first embodiment;
FIG. 10 is a diagram illustrating an arrangement processing unit and a score calculation unit according to a second embodiment; and
FIG. 11 is a diagram illustrating a computer for executing a metabolic analysis program according to the first embodiment.

Preferred embodiments of the present invention will be explained with reference to the accompanying drawings. The metabolic analysis apparatus for executing the metabolic analysis program according to the present invention will now be described using the following embodiments.

### [a] First Embodiment

### Configuration of Metabolic Analysis Apparatus According to First Embodiment

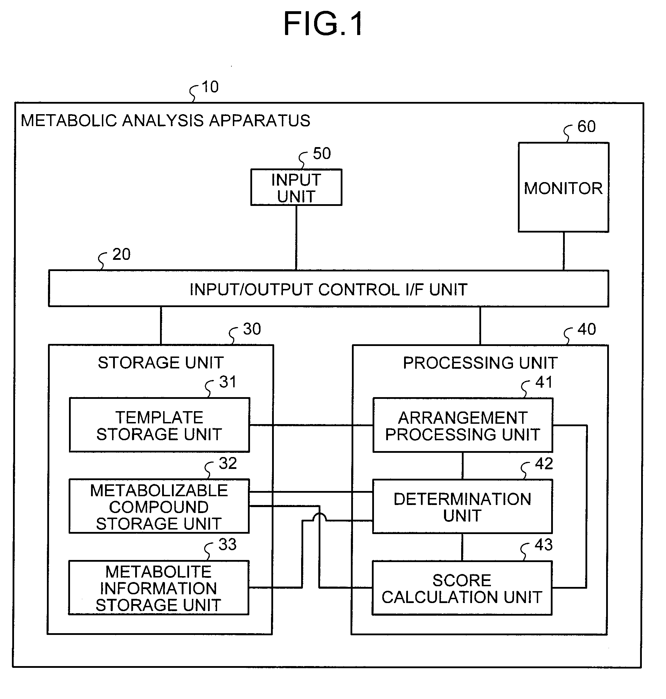

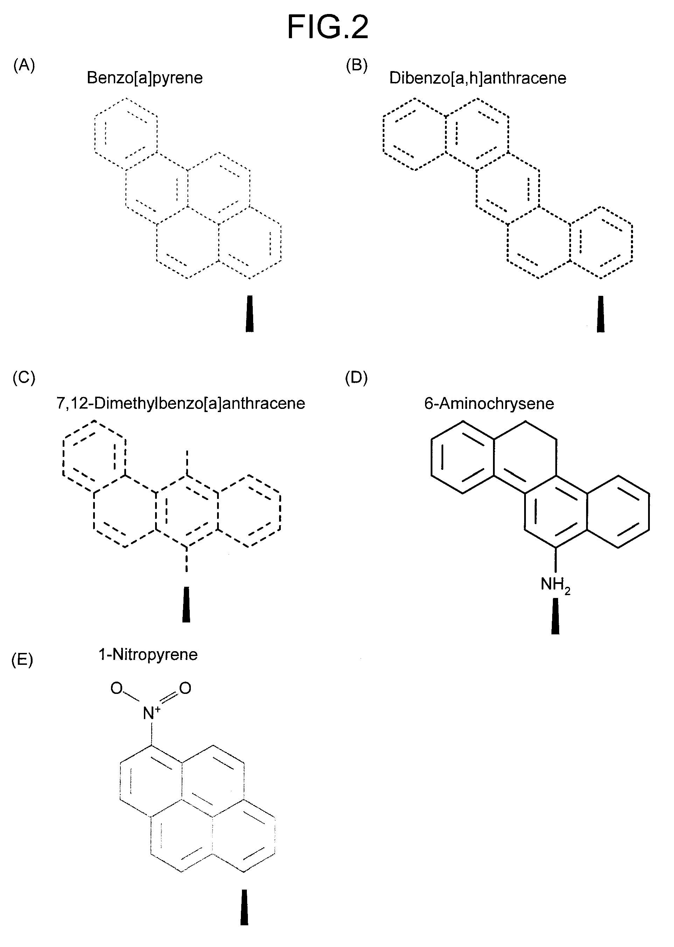

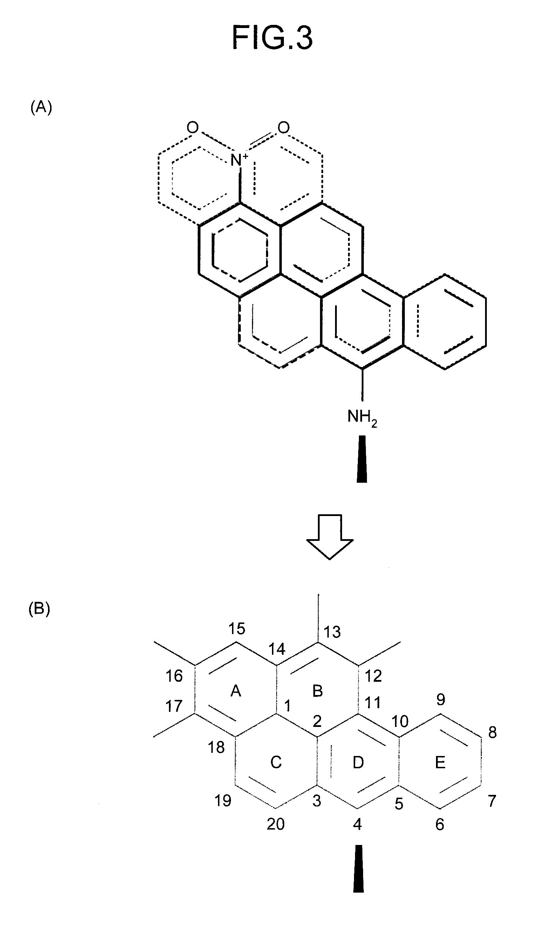

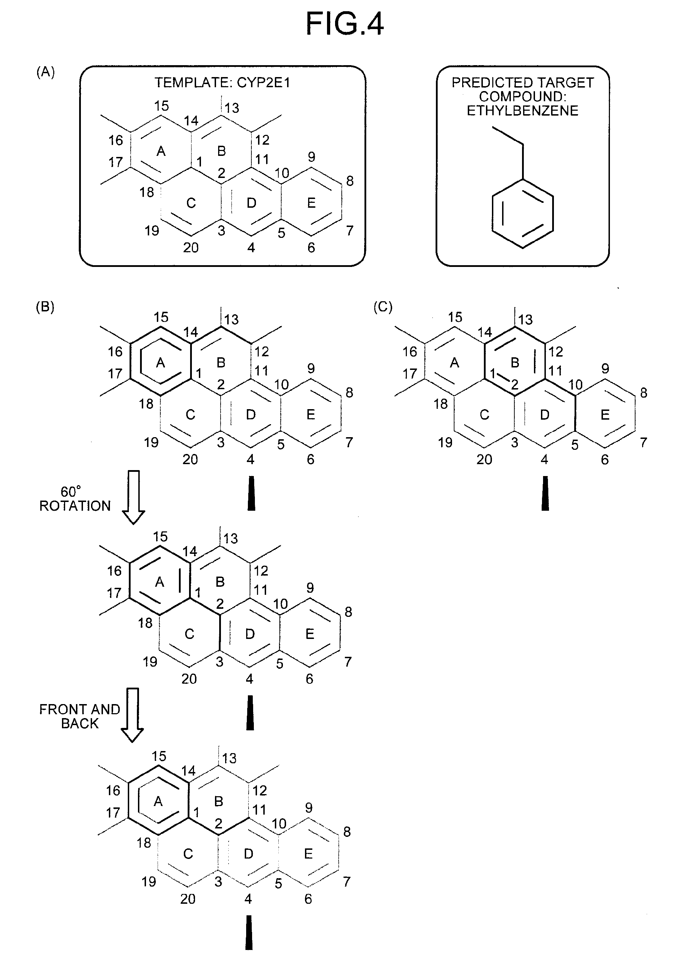

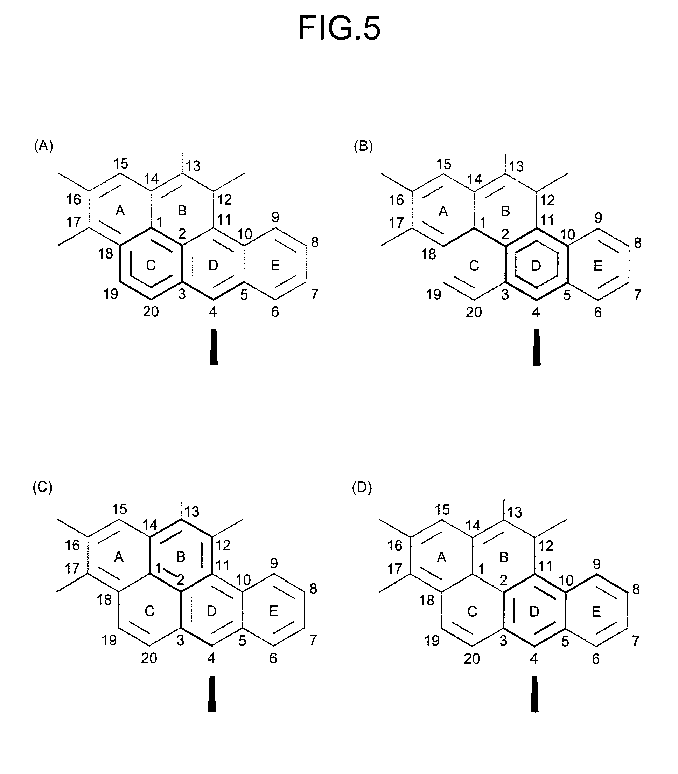

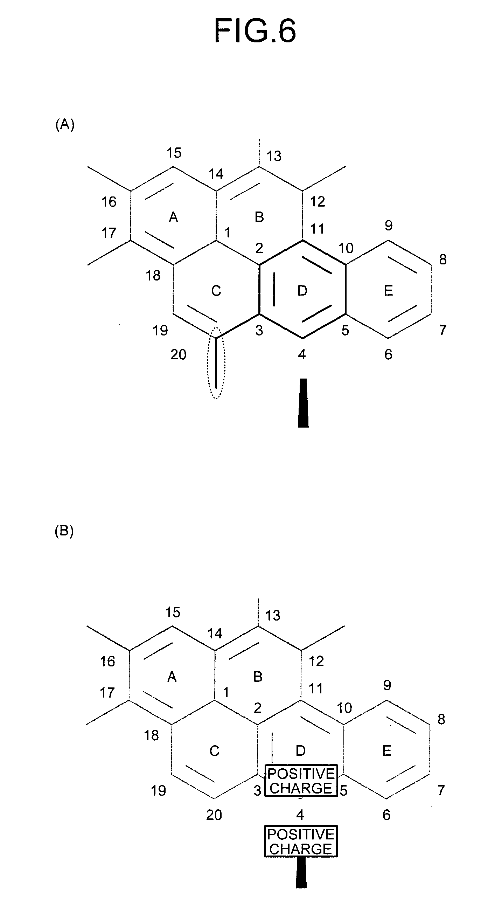

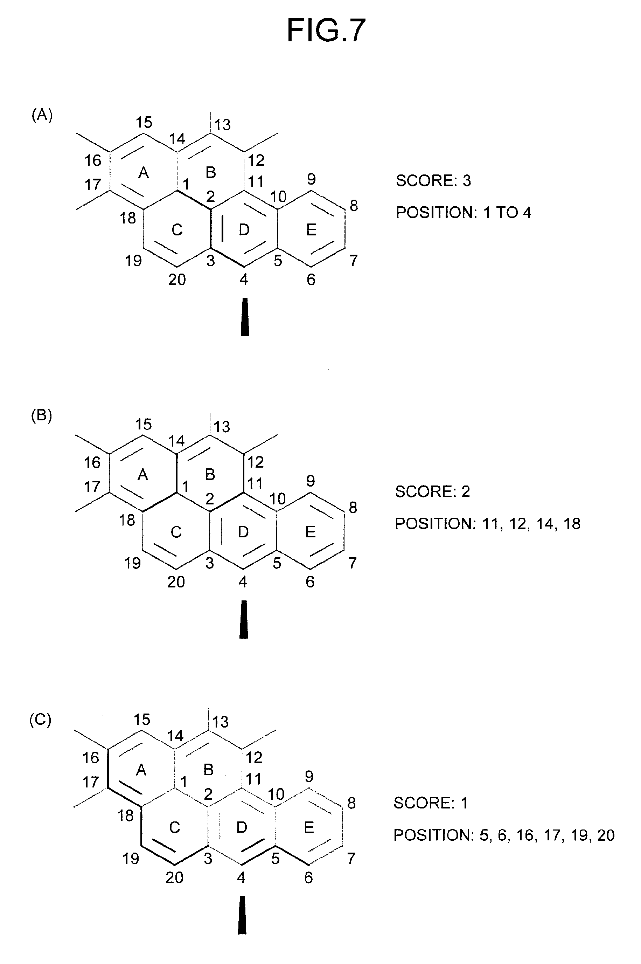

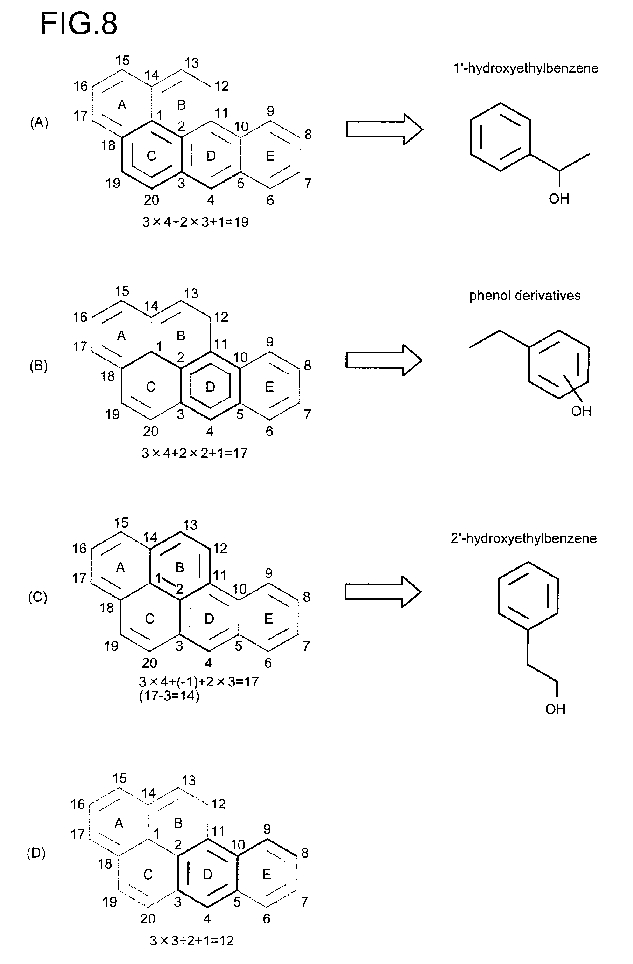

First, the configuration of the metabolic analysis apparatus according to a first embodiment will be described using FIGS. 1 to 8. FIG. 1 is a block diagram illustrating the configuration of the metabolic analysis apparatus according to the first embodiment. FIG. 2 depicts examples of the compound used in template production. FIG. 3 is a diagram illustrating an example of template production. FIG. 4 is a diagram illustrating an example of the arrangement processing unit according to Example 1. FIG. 5 is a diagram illustrating a metabolizable compound. FIG. 6 is a diagram illustrating a constraint. FIG. 7 is a diagram illustrating a score calculation unit. FIG. 8 is a diagram illustrating a score calculation example and a metabolite prediction example according to the first embodiment. In the below, prediction of a metabolic reaction by P450, which is an enzyme group relating to xenobiotic metabolism, will be described for a case in which the prediction is executed by the metabolic analysis apparatus.

As illustrated in FIG. 1, a metabolic analysis apparatus 10 according to the first embodiment has an input/output control I/F unit 20, a storage unit 30, a processing unit 40, an input unit 50, and a monitor 60.

The input/output control I/F unit 20 is an interface which relays data to be sent and received between the storage unit 30, the processing unit 40, the input unit 50, and the monitor 60.

The input unit 50 has a keyboard, a mouse and the like, and receives various information from a user. More specifically, the input unit 50 receives various instructions when the user analyzes a metabolic reaction of a predicted target compound. Further, as an instruction especially closely related to the present embodiment, the input unit 50 receives a two-dimensional structural formula of the predicted target compound generated by the user using a program such as drawing software, for example.

The monitor 60 displays various screens which are referenced by the user. Specifically, the monitor 60 displays a graphical user interface (GUI) during analysis of the metabolic reaction of the predicted target compound, and the results of analysis performed by the processing unit 40.

The storage unit 30 stores various information to be used when the processing unit 40 executes processing, and the results of analysis performed by the processing unit 40. As an example especially closely related to the present embodiment, as illustrated in FIG. 1, the storage unit 30 has a template storage unit 31, a metabolizable compound storage unit 32, and a metabolite information storage unit 33.

The metabolite information storage unit 33 stores metabolite information relating to compounds whose metabolic reactions are already known by various experiments. Specifically, the metabolite information storage unit 33 is a database which associates and stores, as information about known metabolic reactions carried out by the respective members of the P450 enzyme group, information (structural formula) about compounds which serve as a substrate and information (structural formula) about metabolized metabolites.

The template storage unit 31 and the metabolizable compound storage unit 32 will be described in more detail below.

The processing unit 40 executes various processes. As examples especially closely related to the present embodiment, the processing unit 40 has an arrangement processing unit 41, a determination unit 42, and a score calculation unit 43.

The arrangement processing unit 41 arranges the two-dimensional structural formula of the predicted target compound received from the input unit 50 on a template of the P450 designated by the user.

The P450 templates used by the arrangement processing unit 41 will now be described. The P450 templates are stored in the template storage unit 31 in advance. A template is produced for each enzyme belonging to the P450 family from a plurality of compounds which are known to form an enzyme-substrate complex. These templates are stored in the template storage unit 31 by the administrator of the metabolic analysis apparatus 10. More specifically, the administrator of the metabolic analysis apparatus 10 produces the templates by superimposing the two-dimensional structural formulae of a plurality of compounds which are known to form complexes over a metabolic target site. The template production of CYP2E1, which belongs to the P450 family, will now be described below.

The template of CYP2E1 described in the present embodiment can be produced using the five kinds (A) to (E) of compound represented in FIG. 2. The 6-aminochrysene represented in FIG. 2 is a compound which forms a complex with CYP2E1, but is not metabolized by CYP2E1. Therefore, the 6-aminochrysene is an inhibitor which inhibits the metabolic reaction of CYP2E1. The other four compounds represented in FIG. 2 are compounds which form complexes with, and are metabolized by, CYP2E 1. The metabolites formed after the metabolic reaction are also known.

The hexagons illustrated in FIG. 2 represent six-membered rings having not-illustrated carbon atoms arranged at each apex. Further, the double lines in the six-membered rings represent the fact that the bonds between carbon atoms are double bonds. Moreover, the black arrow heads illustrated in FIG. 2 represent, for each compound, the metabolic target site of CYP2E 1. Although a black arrow head is indicated also for the amino group of the 6-aminochrysene illustrated in FIG. 2, this represents that the metabolic reaction of CYP2E 1 is inhibited by the amino group indicated by the black arrow head.

As illustrated in (A) of FIG. 3, during production of the CYP2E1 template, first, the administrator of the metabolic analysis apparatus 10 superimposes the two-dimensional structural formulae of the group of compounds represented in FIG. 2 over the metabolic target site. Then, the administrator produces the template, while considering the states in which the compounds can be arranged at the active pocket of CYP2E1.

For example, the administrator produces the template by considering, for the overlapping group of compounds illustrated in (A) of FIG. 3, moieties having a high degree of overlapping, the position of the metabolic target site, and the fact that the size of the compound metabolizable by CYP2E1 corresponds to five six-membered rings. Consequently, as illustrated in (B) of FIG. 3, the administrator produces a template having five six-membered rings from which two upper six-membered rings were removed. Then, the administrator stores the CYP2E1 template illustrated in (B) of FIG. 3 in the template storage unit 31 via the input unit 50.

The letters and numerals on the template illustrated in (B) of FIG. 3 were provided for the purpose of the following description. The solid lines extending from numerals 12, 13, 16, and 17 represent the fact that a functional group can be added to the atom of the respective numeral. In addition, in FIG. 2, although only five types of compound are illustrated as being used in the template production, in actual practice, the template illustrated in (B) of FIG. 3 is produced from about 100 types of compound.

The arrangement processing unit 41 arranges the predicted target compound on the six-membered rings of the template stored by the template storage unit 31 so that the six-membered ring included in the predicted target compound is at an identical position with the six-membered ring of the template.

A case will now be described in which, as illustrated in (A) of FIG. 4, to predict the metabolic reaction of CYP2E1, which belongs to the P450 family, the user of the metabolic analysis apparatus 10 designates "template: CYP2E1", and inputs "ethylbenzene" as the predicted target compound. First, as illustrated in (B) of FIG. 4, the arrangement processing unit 41 arranges the "six-membered ring of the predicted target compound" on "six-membered ring: A" included in the CYP2E1 template so that it is at an identical position with the "six-membered ring: A".

Then, the arrangement processing unit 41 arranges the predicted target compound so that, with respect to the six-membered rings of the template, the predicted target compound is directed in all of the directions while the six-membered ring of the predicted target compound is arranged at an identical position with the six-membered ring of the template. For example, as illustrated in (B) of FIG. 4, the arrangement processing unit 41 arranges the predicted target compound by rotating it 60□ in a state in which the "six-membered ring of the predicted target compound" is superimposed on "six-membered ring A".

Furthermore, as illustrated in (B) of FIG. 4, the arrangement processing unit 41 inverts the front and back of the predicted target compound, and arranges the predicted target compound in the same manner as above, in which the predicted target compound is directed in all of the directions while the six-membered ring of the predicted target compound is arranged at an identical position with the six-membered ring of the template.

Then, the arrangement processing unit 41 executes rotation arrangement processing on both the front and back of all of the six-membered rings included in the template. For example, when the arrangement processing for the "six-membered ring A" finishes, as illustrated in (C) of FIG. 4, the arrangement processing unit 41 performs arrangement processing on the "six-membered ring: B".

Although the above embodiment has been described for a case in which there is only one six-membered ring included in the predicted target compound, the present embodiment is not limited to this. For example, the above arrangement processing may also be performed when there is a plurality of six-membered rings included in the predicted target compound. If there is a plurality of six-membered rings in the predicted target compound, the arrangement processing unit 41 arranges the predicted target compound so that the plurality of six-membered rings included in the predicted target compound are at identical positions with the plurality of six-membered rings of the template.

When the predicted target compound arranged on the template by the arrangement processing unit 41 overlaps with the metabolic target site of the template in position, the determination unit 42 determines that the arranged predicted target compound is a metabolizable compound which can be metabolized by the enzyme corresponding to the template. More specifically, when the predicted target compound arranged by the arrangement processing unit 41 overlaps with "number: 4", which is the metabolic target site, the determination unit 42 determines that the arranged predicted target compound is a metabolizable compound which can be metabolized by CYP2E 1.

For example, since the predicted target compound overlaps with the metabolic target site "number: 4" of the template in each of the arrangement states illustrated in (A) to (D) of FIG. 5, the determination unit 42 determines that the predicted target compound is a metabolizable compound. In this example, the determination unit 42 considers the atoms of the predicted target compound which overlaps with "number: 4" in each of the arrangement states illustrated in (A) to (D) of FIG. 5 to be metabolic target candidate sites. The score calculation unit 43 performs below-described score calculation processing in order for the determination unit 42 to evaluate the likelihood that an atom serving as a metabolic target candidate site will be metabolized.

Among the predicted target compounds determined as being metabolizable compounds, the determination unit 42 considers the predicted target compounds which satisfy the following constraints as non-metabolizable compounds and excludes them from those to be subjected to the below-described processing. Specifically, the determination unit 42 determines that predicted target compounds determined to be unable to access the metabolic active center site of the enzyme and predicted target compounds having the same charge as that of the metabolic active center site of the enzyme are non-metabolizable compounds. More specifically, the determination unit 42 determines that a predicted target compound is a non-metabolizable compound if the moiety overlapping with the "number: 4" cannot access the metabolic active center of the enzyme due to steric hindrance even when the predicted target compound overlaps with the metabolic target site of the template.

For example, as illustrated in (A) of FIG. 6, when a part of a side chain protrudes from the position of "number: 20", the determination unit 42 determines that the metabolic target site cannot access the active center of the enzyme. Accordingly, the determination unit 42 determines that the predicted target compound is a non-metabolizable compound. Furthermore, although not illustrated, the determination unit 42 performs a similar determination for the "number: 6" position. Specifically, the determination unit 42 excludes a predicted target compound from the metabolizable compounds if the two-dimensional structural formula of the predicted target compound in a state in which the metabolic target site overlaps with the "number: 4" protrudes from the portion below the two-dimensional structural formula (the line between numbers 20, 4, and 6) of the template illustrated in (A) of FIG. 6.

Furthermore, the determination unit 42 determines that a predicted target compound having the same charge as that of the metabolic active center site of the enzyme is a non-metabolizable compound. Also, cases in which a halogen atom is bonded (for example, Cl) are determined to be non-metabolizable compounds. For example, as illustrated in (B) of FIG. 6, when the enzyme active center has a positive charge, and the predicted target compound overlapping with the template also has a positive charge, the determination unit 42 determines that the predicted target compound is a non-metabolizable compound.

Then, the determination unit 42 stores information about the determined metabolizable compounds in the metabolizable compound storage unit 32. For example, the determination unit 42 stores in the metabolizable compound storage unit 32 the respective states (see (A) to (D) of FIG. 5) in which the ethylbenzene determined as being a metabolizable compound was arranged with respect to the template.

The score calculation unit 43 calculates a score which indicates the likelihood that a metabolic target site of a metabolizable compound in the state as arranged by the arrangement processing unit 41 will be metabolized by the enzyme based on a level of contribution of each atom in the template during an enzyme metabolic reaction. More specifically, the score calculation unit 43 calculates a score based on whether each atom of the metabolizable compound stored by the metabolizable compound storage unit 32 is arranged at the same position as an atom which contributes during the metabolic reaction to structural stability, to bonding with the enzyme, and to the metabolic target site accessing to the active center.

For example, as illustrated in (A) of FIG. 7, since the atoms arranged at positions "number: 1" to "number: 4" contribute to the metabolic target site accessing to the active center, the score calculation unit 43 gives a "score: 3" to each atom. Further, as illustrated in (B) of FIG. 7, since the atoms arranged at positions "number: 11", "number: 12", "number: 14", and "number: 18" contribute to bonding with the enzyme, the score calculation unit 43 gives a "score: 2" to each atom. Still further, as illustrated in (C) of FIG. 7, since the atoms arranged at positions "number: 5", "number: 6", "number: 16", "number: 17", "number: 19", and "number: 20" contribute to structural stability during the metabolic reaction, the score calculation unit 43 gives a "score: 1" to each atom.

Further, the score calculation unit 43 adds to or subtracts from the calculated score based on whether a lone electron pair or a pi bond is included in the metabolizable compound. Specifically, the score calculation unit 43 adds to or subtracts from the calculated score based on the structural stability of the metabolizable compound itself. For example, if a lone electron pair or a pi bond is not included in the atoms arranged at positions "number: 1" to "number: 4", which have a "score: 3", since stability is low, the score calculation unit 43 subtracts one from the score of these atoms, to give them a score of "2".

If a lone electron pair or a pi bond is included in the ring structure to which the atoms arranged at positions "number: 16", "number: 17", "number: 19", and "number: 20", which have a "score: 1", are bonded, since stability is high, the score calculation unit 43 adds one to the score of these atoms, to give them a score of "2".

The score calculation unit 43 gives such a score to each atom in the metabolizable compound arranged in the template, and calculates the total of the given scores. For example, if the metabolizable compound is in the state illustrated in (A) of FIG. 5, as illustrated in (A) of FIG. 8, the score calculation unit 43 calculates the score indicating the likelihood that the metabolic target site will be metabolized by the enzyme as "3 x 4 + 2 x 3 + 1 = 19". Here, the "3 x 4" represents the fact that there are "four" (numbers 1 to 4) atoms having a score of "3", "2 x 3" represents the fact that there are "three" (numbers 18, 19, and 20) atoms having a score of "2", and "1" represents the fact that there is "one" (number 5) atom having a score of "1". Further, since numbers "19" and "20" have a pi bond in the ring structure, "1" is added to the score of "1" to give a score of "2".

In addition, if the metabolizable compound is in the states illustrated in FIGS. 5B, 5C, and 5D, as illustrated in FIGS. 8B, 8C, and 8D, the score calculation unit 43 calculates the score indicating the likelihood that each metabolic target site will be metabolized by CYP2E1. Specifically, for the state illustrated in FIG. 5B, the score calculation unit 43 calculates the score as "3 x 4 + 2 x 2 + 1 = 17". Further, for the state illustrated in FIG. 5C, the score calculation unit 43 calculates the score as "3 x 4 + (-1) + 2 x 3 = 17". For the state illustrated in FIG. 5D, the score calculation unit 43 calculates the score as "3 x 3 + 2 + 1 = 12".

Next, the determination unit 42 determines priority based on the fact that the higher the score calculated by the score calculation unit 43, the higher the likelihood that the metabolic target site in each arrangement state of the metabolizable compound will be metabolized by CYP2E 1. For example, regarding the metabolic target site of the respective arrangement states illustrated in FIG. 8, the determination unit 42 determines the priority in the order of "(A), (B), (C), and (D)" based on higher likelihood of being metabolized by CYP2E 1.

Then, based on the priority, the determination unit 42 narrows down the respective arrangement states of the metabolizable compound for predicting the metabolites. More specifically, the determination unit 42 predicts metabolites only for the metabolic target site in arrangement states which have a difference with the highest score of less than 3 ((A), (B), and (C) of FIG. 8). For example, the determination unit 42 determines the metabolites by predicting the reaction for when each of the metabolic target sites of the arrangement states illustrated in (A), (B), and (C) of FIG. 8 is metabolized by CYP2E1.

More specifically, the determination unit 42 refers to the metabolite information stored by the metabolite information storage unit 33 to predict the metabolic reaction, and determines the metabolites. For example, the determination unit 42 determines that "1'-hydroxyethylbenzene" is a metabolite in which the metabolic target site of the ethylbenzene in the arrangement state illustrated in (A) of FIG. 8 was hydroxylated.

Further, the determination unit 42 determines that "phenyl derivatives" derived by hydroxylating the metabolic target site of the ethylbenzene in the arrangement state illustrated in (B) of FIG. 8 are metabolites. Still further, the determination unit 42 determines that "2'-hydroxyethylbenzene" is a metabolite in which the metabolic target site of the ethylbenzene in the arrangement state illustrated in (C) of FIG. 8 was hydroxylated.

In addition, the determination unit 42 determines that metabolizable compounds for which there is no information in the metabolite information about the metabolic reaction are inhibitors.

Further, the score calculation unit 43 may also subtract from the calculated score based on the overall arrangement state of the metabolizable compound with respect to the template. More specifically, in the arrangement state of the metabolizable compound with respect to the template, if there is no atom arranged at "number: 5", "number: 6", "number: 16", "number: 17", "number: 19", or "number: 20", which contributes to structural stability during the metabolic reaction, the score calculation unit 43 may subtract from the score.

For example, if there is no atom arranged at a position which contributes to structural stability during the metabolic reaction, "3" is subtracted from the calculated score of "17", to give a final score of "14" (see the equation inside the brackets in (C) of FIG. 8).

If the final score of the metabolizable compound in the arrangement state illustrated in (C) of FIG. 8 is "14", since the final score is lower than the highest value of"19" by "3" or more, the determination unit 42 excludes the metabolic target site of (C) of FIG. 8 from the determined target metabolites.

The metabolizable compounds illustrated in (A) to (D) of FIG. 5 are merely examples. In reality, many more metabolizable compounds exist. Further, although the above-described embodiment was described using CYP2E1 as the template, the present exemplary embodiment is not limited to this. Other enzymes may also be used.

### Sequence of Metabolite Determination Processing by Metabolic Analysis Apparatus According to First Embodiment

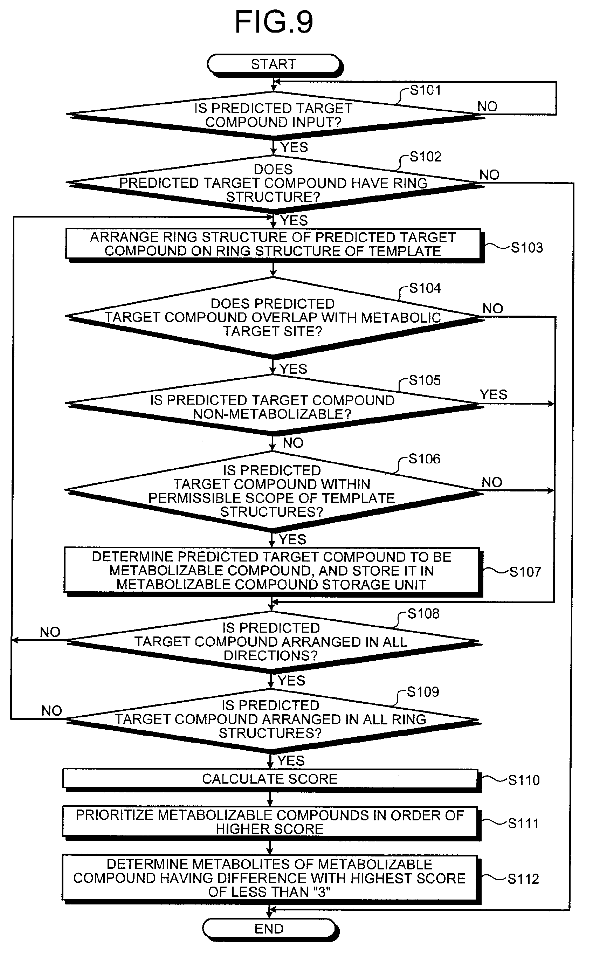

Next, with reference to FIG. 9, the metabolite determination processing performed by the metabolic analysis apparatus according to the first embodiment will be described. FIG. 9 is a diagram illustrating a sequence of metabolite determination processing performed by the metabolic analysis apparatus according to the first embodiment.

As illustrated in FIG. 9, when a predicted target compound is input into the metabolic analysis apparatus 10 according to the first embodiment along with the designation of the template by a user (YES at step S 101), the arrangement processing unit 41 determines whether there is a ring structure in the predicted target compound input by the user (step S102). If it is determined that there is no ring structure in the predicted target compound input by the user (NO at step S102), the arrangement processing unit 41 finishes the processing.

On the other hand, if it is determined that there is a ring structure in the predicted target compound input by the user (YES at step S 102), the arrangement processing unit 41 arranges the ring structure of the predicted target compound so as to be at an identical position with the ring structure in the template of the enzyme designated by the user (step S103). More specifically, the arrangement processing unit 41 arranges the six-membered ring of the predicted target compound so as to be at an identical position with the six-membered ring included in the template.

Then, if the predicted target compound overlaps with the metabolic target site (YES at step S104), the determination unit 42 determines whether the predicted target compound is non-metabolizable (step S105). More specifically, the determination unit 42 determines whether the predicted target compound has the same charge as that of the metabolic active center site of the enzyme. For example, if the enzyme active center site has a positive charge, and the predicted target compound overlapping with the template also has a positive charge, the determination unit 42 determines that the predicted target compound is a non-metabolizable compound (see (B) of FIG. 6).

If the predicted target compound is metabolizable (NO at step S 105), the determination unit 42 determines whether the predicted target compound is within a permissible scope of template structures (step S 106). More specifically, the determination unit 42 determines whether the moiety of the predicted target compound overlapping with the metabolic target site can access the enzyme metabolic active center in view of steric hindrance (see FIG. 6A).

If the predicted target compound is within the permissible scope of template structures (YES at step S 106), the determination unit 42 determines that the predicted target compound is a metabolizable compound, and stores in the metabolizable compound storage unit 32 the metabolizable compound in the state arranged in the template (step S107).

On the other hand, if the predicted target compound is not within the permissible scope of template structures (NO at step S106), or if the predicted target compound is non-metabolizable (YES at step S105), or if the predicted target compound does not overlap with the metabolic target site of the template (NO at step S104), or if the processing of step S107 has finished, the determination of step S 108 is carried out. Specifically, the arrangement processing unit 41 determines whether the six-membered ring of the predicted target compound was arranged on the six-membered ring of the template in all of the directions that it can be arranged at an identical position with the six-membered ring of the template.

If the predicted target compound was not arranged in all directions (NO at step S108), the arrangement processing unit 41 returns the processing to step S103, and arranges the six-membered ring of the predicted target compound on the six-membered ring of the template in an unprocessed direction.

On the other hand, if the predicted target compound was arranged in all directions (YES at step S108), the arrangement processing unit 41 determines whether the predicted target compound was arranged on all ring structures (step S109). More specifically, the arrangement processing unit 41 determines whether the predicted target compound was arranged on all of the six-membered rings included in the template.

If the predicted target compound was not arranged on all of the ring structures (NO at step S109), the arrangement processing unit 41 arranges the six-membered ring of the predicted target compound on an unprocessed six-membered ring of the template.

On the other hand, if the predicted target compound was arranged on all of the ring structures (YES at step S109), the score calculation unit 43 calculates the score of the metabolizable compound stored by the metabolizable compound storage unit 32 (step S110). More specifically, the score calculation unit 43 calculates a score indicating the likelihood that a metabolic target site of the metabolizable compound will be metabolized by the enzyme based on whether each atom of the metabolizable compound is arranged at the same position as an atom which contributes during the metabolic reaction to structural stability, to bonding with the enzyme, and to the metabolic target site accessing to the active center.

Here, the score calculation unit 43 adds to or subtracts from the calculated score based on whether a lone electron pair or a pi bond is included in the metabolizable compound. Specifically, the score calculation unit 43 adds to or subtracts from the calculated score based on the structural stability of the metabolizable compound itself.

Then, the determination unit 42 prioritizes the metabolic target sites of the metabolizable compounds in order of higher score (step S111).

Next, the determination unit 42 refers to the metabolite information stored by the metabolite information storage unit 33 and determines the metabolites of a metabolizable compound having a difference with the highest score of less than "3" (step S 112), and finishes the processing. If there is no information relating to the metabolic reaction in the metabolite information, the determination unit 42 determines that metabolizable compounds for which there is no information about the metabolic reaction are inhibitors which inhibit the metabolic reaction.

### Effects of First Embodiment

As described above, according to the first embodiment, the arrangement processing unit 41 arranges a predetermined structure of a predicted target compound which is in a two-dimensional structural formula so as to be in an identical position with a predetermined structure of a template produced by superimposing the two-dimensional structural formulae of a plurality of compounds capable of forming a complex with P450. Then, if the two-dimensional structural formula of the predicted target compound arranged on the template by the arrangement processing unit 41 overlaps with a metabolic target site of P450 in the template, the determination unit 42 determines that the predicted target compound is a metabolizable compound which can be metabolized by P450. Therefore, whether a compound will be metabolized can be determined using the two-dimensional structural formula of the compound, and the metabolic reactions can be easily predicted.

Furthermore, according to the first embodiment, the determination unit 42 determines that, of the predicted target compounds determined to be metabolizable compounds, predicted target compounds determined as being unable to access the P450 metabolic active center site and predicted target compounds having the same charge as that of the metabolic active center site are non-metabolizable compounds. Therefore, sites which will not be metabolized can be excluded, and the metabolic reactions can be predicted more easily.

Moreover, according to the first embodiment, the score calculation unit 43 calculates a score which indicates the likelihood that a metabolic target site of a metabolizable compound in a state arranged with respect to the template by the arrangement processing unit 41 will be metabolized by P450 based on the level of contribution of each atom included in the template during the P450 metabolic reaction. In addition, the determination unit 42 determines the metabolic target sites of the metabolizable compounds based on a score calculated by the score calculation unit 43. Therefore, the priority of the metabolic target sites in the metabolizable compounds, which conventionally could not be determined, can be determined, and the metabolic reactions can be predicted even more easily.

Further, according to the first embodiment, based on information about metabolites produced by P450, the determination unit 42 determines the structural formula of a compound obtained after metabolic reaction of the metabolizable compound whose metabolic target site was determined. Therefore, easily produced metabolites can be determined, and highly accurate prediction is possible.

Moreover, according to the first embodiment, if there is no information about the compound obtained after the metabolic reaction of the metabolizable compound whose metabolic target site was determined in the information about the metabolites produced by P450, the determination unit 42 determines that metabolizable compounds for which there is no information about the compound are inhibitors which inhibit the metabolic reaction. Therefore, the specific properties of a metabolizable compound in the metabolic reaction can be analyzed in detail, and prediction can be carried out more accurately.

Furthermore, according to the first embodiment, the score calculation unit 43 calculates a score based on whether each atom of the metabolizable compound in a state arranged with respect to the template by the arrangement processing unit 41 is arranged at the same position as an atom which contributes to structural stability during the metabolic reaction. In addition, the score calculation unit 43 calculates the score based on whether each atom of the metabolizable compound in a state arranged with respect to the template by the arrangement processing unit 41 is arranged at the same position as an atom which contributes to bonding with P450 during the metabolic reaction, and whether each atom of the metabolizable compound is arranged at the same position as an atom which contributes to the metabolic target site accessing to the active center of P450 during the metabolic reaction. Therefore, the metabolic site can be determined and prediction can be carried out more accurately by considering the states in which the metabolizable compound can be easily arranged in the P450 active pocket.

Moreover, according to the first embodiment, the score calculation unit 43 adds to or subtracts from the calculated score based on whether a lone electron pair or a pi bond is included in the metabolizable compound. Therefore, the metabolic site can be determined based on the structural stability and reactivity of the metabolizable compound, and prediction can be carried out even more accurately.

### [b] Second Embodiment

In the above first embodiment, a case was described in which a six-membered ring of the predicted target compound was arranged on a six-membered ring of the template. In a second embodiment, a case will be described in which a five-membered ring included in the predicted target compound is arranged on a six-membered ring of the template.

### Configuration of Metabolic Analysis Apparatus According to Second Embodiment

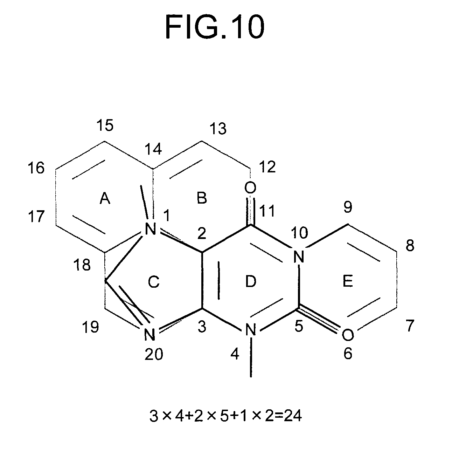

First, the configuration of the metabolic analysis apparatus according to the second embodiment will be described using FIGS. 1 and 10. FIG. 1 is a block diagram illustrating the configuration of the metabolic analysis apparatus according to the first embodiment. FIG. 10 is a diagram illustrating an arrangement processing unit and a score calculation unit according to the second embodiment. The metabolic analysis apparatus 10 in the second embodiment has similar blocks as the metabolic analysis apparatus in the first embodiment. However, the processing details of the arrangement processing unit 41 and the score calculation unit 43 are different from the first embodiment. the second embodiment will be described below focusing on these differences.

If a structure similar to a predetermined structure is included in the two-dimensional structural formula of the predicted target compound, the arrangement processing unit 41 arranges the predicted target compound on the template at a position of the template having the maximum degree of overlapping between the predetermined structure and the structure similar thereto. More specifically, if a ring structure other than a six-membered ring is included in the predicted target compound, the arrangement processing unit 41 arranges the predicted target compound at a position having the maximum degree of overlapping between the six-membered ring of the template and the ring structure other than a six-membered ring of the predicted target compound (for example, a five-membered ring or a seven-membered ring).

For example, as illustrated in FIG. 10, the arrangement processing unit 41 arranges the predicted target compound at a position having the maximum degree of overlapping between the six-membered ring of the template and the five-membered ring of the predetermined target compound. For the metabolic target site "number: 4" in the CYP2E1 template, even if one methyl group is added, the added methyl group does not sterically hinder the metabolic target site from accessing the active center.

The score calculation unit 43 gives the appropriate score to the atoms arranged on the dark line portion illustrated in ( A ) to (C) of FIG. 7 based on the score calculation of the metabolizable compound determined by the determination unit 42. For example, as illustrated in FIG. 10, the score calculation unit 43 gives a score of "2" to the atoms of the five-membered ring arranged along the line connecting "number: 18" and "number: 19". This score of "2" was calculated by adding "1" to the score of "1", since there is a pi bond in the ring structure.

Specifically, the score calculation unit 43 calculates the score for the metabolizable compound illustrated in FIG. 10 as "3 x 4 + 2 x 5 + 1 x 2 = 24". Regarding the processing other than this, such processing is the same as that described in the first embodiment, and thus a description thereof is omitted here.

### Sequence of Metabolite Determination Processing by Metabolic Analysis Apparatus According to Second Embodiment

Other than the method for the arrangement processing of the predicted target compound having a ring structure similar to the ring structure of the template, and the method for the score calculation processing, the metabolite determination processing performed by the metabolic analysis apparatus according to the second embodiment is the same as that performed in the first embodiment which was described with reference to FIG. 9. Thus, a description thereof is omitted here.

### Effects of Second Embodiment

As described above, according to the second embodiment, if a structure similar to P450 is included in the two-dimensional structural formula of the predicted target compound, the arrangement processing unit 41 arranges the predicted target compound on the template at a position having the maximum degree of overlapping between the predetermined structure of the template and the structure similar thereto. Therefore, even for a predicted target compound having a ring structure other than the ring structure which the template has, the metabolites and metabolic reactions can be easily predicted.

### [c] Other Embodiments

Although the first and the second embodiments have been described above, the present invention may be embodied in various other modes other than the first and the second embodiments. Accordingly, various different embodiments will now be divided into items (1) to (5) and described below.

### (1) Template and Predicted Target Compound Structure

In the above-described embodiments, cases were described in which the same or a similar ring structure in the template and the predicted target compound was superimposed. However, the present embodiment is not limited to this. For example, a chain structure of a predicted target compound may be superimposed over a chain structure which a template has.

### (2) Metabolic Analysis Target Enzyme

In the above-described embodiments, cases were described in which a metabolic reaction catalyzed by CYP2E1, which belongs to the P450 family, is analyzed. However, the present embodiment is not limited to this. For example, analysis may be performed on a metabolic reaction catalyzed by some other member of the P450 family, such as CYP1A2, or by a metabolic enzyme other than P450.

### (3) Modification

In the above-described embodiments, cases were described in which the metabolic analysis was performed with the metabolic analysis apparatus being a standalone piece of equipment. However, the present embodiment is not limited to this. For example, the metabolic analysis apparatus may be installed in a computer which controls an analyzer such as a mass spectrophotometer used in identification of known substances and structural determination of unknown substances.

### (4) System Configuration etc.

Further, the processing sequence, specific names, and information including the various data and parameters described in the above embodiments may be arbitrarily updated unless otherwise noted. For example, the enzyme template in the first and the second embodiments may be arbitrarily updated by the administrator.

In addition, the various structural elements of the various illustrated apparatuses are functional concepts, and do not have to be identical to the structural elements which were physically illustrated. Specifically, the specific mode (for example, the mode of FIG. 1) of how the various processing units and the various storage units are distributed or integrated is not limited to that illustrated. For example, the metabolite information storage unit 33 may be arranged externally to the metabolic analysis apparatus 10. Moreover, all or an arbitrary part of the various processing functions performed by the various apparatuses may be realized by a CPU or a program which is executed by such a CPU, or realized as hardware based on wired logic.

### (5) Metabolic Analysis Program

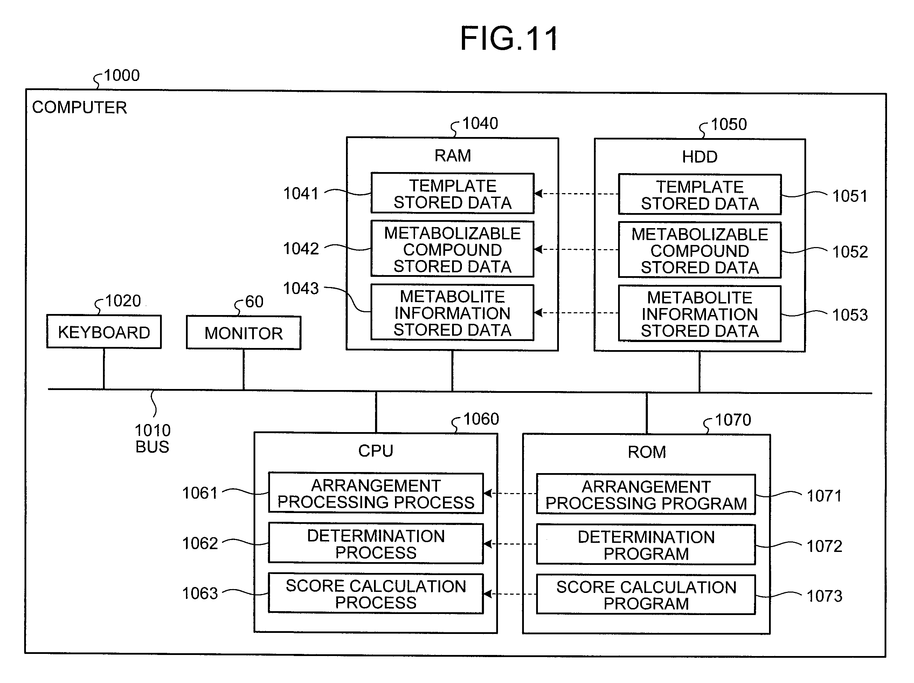

In the above first and second embodiments, cases were described in which the various processes are realized by hardware logic. However, the present embodiment is not limited to this. The various process may also be realized by having a computer execute a prepared program. Accordingly, using FIG. 11, an example of a computer for executing a metabolic analysis program having the same functions as the metabolic analysis apparatus 10 illustrated in the first embodiment will now be described below. FIG. 11 is a diagram illustrating a computer for executing a metabolic analysis program according to the first embodiment.

As illustrated in FIG. 11, a computer 1000 serving as an information processing apparatus has a keyboard 1020, the monitor 60, a RAM 1040, an HDD 1050, a CPU 1060, and a ROM 1070. The keyboard 1020, the monitor 60, the RAM 1040, the HDD 1050, the CPU 1060, and the ROM 1070 are connected by a bus 1010 and the like.

Metabolic analysis programs, specifically, as illustrated in FIG. 11, an arrangement processing program 1071, a determination program 1072, and a score calculation program 1073, for exercising the same functions as the metabolic analysis apparatus 10 illustrated in the above described the first embodiment, are pre-stored in the ROM 1070. Like the various structural elements of the metabolic analysis apparatus 10 illustrated in FIG. 1, these programs 1071 to 1073 may be appropriately integrated or distributed.

Further, by having the CPU 1060 read and execute these programs 1071 to 1073 from the ROM 1070, as illustrated in FIG. 11, the programs 1071 to 1073 function as an arrangement processing process 1061, a determination process 1062, and a score calculation process 1063. The processes 1061 to 1063 correspond to the arrangement processing unit 41, the determination unit 42, and the score calculation unit 43 illustrated in FIG. 1, respectively.

Further, as illustrated in FIG. 11, template stored data 1051, metabolizable compound stored data 1052, and metabolite information stored data 1053 are provided in the HDD 1050. The template stored data 1051, the metabolizable compound stored data 1052, and the metabolite information stored data 1053 correspond to the template storage unit 31, the metabolizable compound storage unit 32, and the metabolite information storage unit 33 used in FIG. 1, respectively. In addition, the CPU 1060 registers template stored data 1041, metabolizable compound stored data 1042, and metabolite information stored data 1043 for the template stored data 1051, the metabolizable compound stored data 1052, and the metabolite information stored data 1053. The CPU 1060 also reads the template stored data 1041, the metabolizable compound stored data 1042, and the metabolite information stored data 1043 and stores the data in the RAM 1040. Still further, the CPU 1060 executes metabolite analysis processing based on the template stored data 1041, the metabolizable compound stored data 1042, and the metabolite information stored data 1043 stored in the RAM 1040.

The respective programs 1071 to 1073 are not necessarily stored in the ROM 1070 from the beginning. The programs 1071 to 1073 may be stored in, for example, a "portable physical medium" which is installed into the computer 1000 such as a flexible disk (FD), a CD-ROM, a magneto-optical disk, a DVD, and an IC card, a "fixed physical medium" such as a HDD that is installed inside or outside of the computer 1000, or "another computer (or a sever)" that is connected to the computer 1000 through a public line, the Internet, a LAN, or a WAN. Each of these programs may be executed by having the computer 1000 read them.

The described apparatus enables easy prediction of a metabolic reaction.

## Claims

1. A metabolic analysis program causing a computer to execute a process comprising:
arranging a predetermined structure of a predicted target compound which is in a two-dimensional structural formula so as to be in an identical position with a predetermined structure of a template produced by superimposing two-dimensional structural formulae of a plurality of compounds capable of forming a complex with a predetermined enzyme; and
determining, if the two-dimensional structural formula of the predicted target compound arranged on the template by the arranging overlaps with a metabolic target site of the predetermined enzyme in the template, that the predicted target compound is a metabolizable compound which can be metabolized by the predetermined enzyme.

2. The program according to claim 1, wherein the determining includes determining that, among predicted target compounds determined as being the metabolizable compounds, predicted target compounds determined as being unable to access a metabolic active center site of the predetermined enzyme and predicted target compounds having the same charge as that of the metabolic active center site of the predetermined enzyme are compounds which cannot be metabolized by the predetermined enzyme.

3. The program according to claim 1 or 2, wherein the metabolic analysis program further causes the computer to execute calculating an index indicating a likelihood that a metabolic target site of the metabolizable compound in a state arranged on the template by the arranging will be metabolized by the predetermined enzyme based on a level of contribution of each atom included in the template during a metabolic reaction of the predetermined enzyme, and wherein
the determining includes determining the metabolic target site of the metabolizable compound based on the index calculated by the calculating.

4. The program according to claim 3, wherein the determining includes determines a structural formula of a compound obtained after the metabolic reaction of the metabolizable compound whose metabolic target site has been determined based on information about metabolites produced by the predetermined enzyme.

5. The program according to claim 4, wherein the determining includes determining that the metabolizable compound is an inhibitor which inhibits the metabolic reaction when there is no information about a compound obtained after the metabolic reaction of the metabolizable compound whose metabolic target site has been determined in the information about the metabolites produced by the predetermined enzyme.

6. The program according to claim 3, wherein the calculating includes calculating the index based on whether each atom of the metabolizable compound in a state arranged on the template by the arranging is arranged at the same position as an atom contributing to structural stability during the metabolic reaction, whether each atom of the metabolizable compound is arranged at the same position as an atom contributing to bonding with the predetermined enzyme during the metabolic reaction, and whether each atom of the metabolizable compound is arranged at the same position as an atom contributing to the metabolic target site accessing to the active center of the predetermined enzyme during the metabolic reaction.

7. The program according to claim 6, wherein the calculating includes adding to or subtracting from the calculated index based on whether a lone electron pair or a pi bond is included in the metabolizable compound.

8. The program according to any preceding claim, wherein, when a structure similar to the predetermined structure is included in the two-dimensional structural formula of the predicted target compound, the arranging includes arranging the predicted target compound on the template at a position having the maximum degree of overlapping between the predetermined structure of the template and the similar structure.

9. A metabolic analysis apparatus, comprising:
an arrangement processing unit (41) configured to arrange a predetermined structure of a predicted target compound which is in a two-dimensional structural formula so as to be in an identical position with a predetermined structure of a template produced by superimposing two-dimensional structural formulae of a plurality of compounds capable of forming a complex with a predetermined enzyme; and
a determination unit (42) configured to determine, if the two-dimensional structural formula of the predicted target compound arranged on the template by the arrangement processing unit (41) overlaps with a metabolic target site of the predetermined enzyme in the template, that the predicted target compound is a metabolizable compound which can be metabolized by the predetermined enzyme.

10. The metabolic analysis apparatus according to claim 9, wherein the determination unit (42) is arranged to determine that, among predicted target compounds determined as being the metabolizable compounds, predicted target compounds determined as being unable to access a metabolic active center site of the predetermined enzyme and predicted target compounds having the same charge as that of the metabolic active center site of the predetermined enzyme are compounds which cannot be metabolized by the predetermined enzyme.

11. The metabolic analysis apparatus according to claim 9 or 10, further comprising an index calculation unit (43) configured to calculate an index indicating a likelihood that a metabolic target site of the metabolizable compound in a state arranged on the template by the arrangement processing unit (41) will be metabolized by the predetermined enzyme based on a level of contribution of each atom included in the template during a metabolic reaction of the predetermined enzyme, and wherein
the determination unit (42) is arranged to determine the metabolic target site of the metabolizable compound based on the index calculated by the index calculation unit (43).

12. A metabolic analysis method, comprising:
arranging a predetermined structure of a predicted target compound which is in a two-dimensional structural formula so as to be in an identical position with a predetermined structure of a template produced by superimposing two-dimensional structural formulae of a plurality of compounds capable of forming a complex with a predetermined enzyme; and
determining, if the two-dimensional structural formula of the predicted target compound arranged on the template by the arranging overlaps with a metabolic target site of the predetermined enzyme in the template, that the predicted target compound is a metabolizable compound which can be metabolized by the predetermined enzyme.

13. The metabolic analysis method according to claim 12, wherein the determining includes determining that, among predicted target compounds determined as being the metabolizable compounds, predicted target compounds determined as being unable to access a metabolic active center site of the predetermined enzyme and predicted target compounds having the same charge as that of the metabolic active center site of the predetermined enzyme are compounds which cannot be metabolized by the predetermined enzyme.

14. The metabolic analysis method according to claim 12 or 13, further comprising calculating an index indicating a likelihood that a metabolic target site of the metabolizable compound in a state arranged on the template by the arranging will be metabolized by the predetermined enzyme based on a level of contribution of each atom included in the template during a metabolic reaction of the predetermined enzyme, and wherein
the determining includes determining the metabolic target site of the metabolizable compound based on the index calculated by the calculating.

## Amended claims

### Amended claims in accordance with Rule 137(2) EPC.

**1.** A metabolic analysis program causing a computer to execute a process comprising:
arranging a predetermined structure of a predicted target compound which is in a structural formula so as to be in an identical position with a predetermined structure of a template produced by superimposing structural formulae of a plurality of compounds capable of forming a complex with a predetermined enzyme; and
determining, if the structural formula of the predicted target compound arranged on the template by the arranging overlaps with a metabolic target site of the predetermined enzyme in the template, that the predicted target compound is a metabolizable compound which can be metabolized by the predetermined enzyme; **characterised in that**:
the structural formulae of the predicted target compound and of said plurality of compounds used to produce said template are two-dimensional structural formulae;
the arranging and determining are performed for each of a plurality of arrangement states of the predicted target compound with respect to the template; and further **characterised by**:
calculating a score indicating a likelihood that the metabolic target site of the metabolizable compound determined by the determining in each arrangement state will be metabolized by the predetermined enzyme based on a level of contribution of each atom included in the template during a metabolic reaction of the predetermined enzyme; and
prioritizing the arrangement states in order of higher score calculated by the calculating, and predicting metabolites of the metabolizable compound only for the metabolic target site in arrangement states having a difference from the highest score within a predetermined range.

**2.** The program according to claim 1, wherein the determining includes determining that, among predicted target compounds determined as being the metabolizable compounds, predicted target compounds determined as being unable to access a metabolic active center site of the predetermined enzyme and predicted target compounds having the same charge as that of the metabolic active center site of the predetermined enzyme are compounds which cannot be metabolized by the predetermined enzyme.

**3.** The program according to claim 1, wherein the determining includes determining a structural formula of a compound obtained after the metabolic reaction of the metabolizable compound whose metabolic target site has been determined based on information about metabolites produced by the predetermined enzyme.

**4.** The program according to claim 3, wherein the determining includes determining that the metabolizable compound is an inhibitor which inhibits the metabolic reaction when there is no information about a compound obtained after the metabolic reaction of the metabolizable compound whose metabolic target site has been determined in the information about the metabolites produced by the predetermined enzyme.

**5.** The program according to claim 3, wherein the calculating includes calculating the score based on whether each atom of the metabolizable compound in a state arranged on the template by the arranging is arranged at the same position as an atom contributing to structural stability during the metabolic reaction, whether each atom of the metabolizable compound is arranged at the same position as an atom contributing to bonding with the predetermined enzyme during the metabolic reaction, and whether each atom of the metabolizable compound is arranged at the same position as an atom contributing to the metabolic target site accessing to the active center of the predetermined enzyme during the metabolic reaction.

**6.** The program according to claim 5, wherein the calculating includes adding to or subtracting from the calculated index based on whether a lone electron pair or a pi bond is included in the metabolizable compound.

**7.** The program according to any preceding claim, wherein, when a structure similar to the predetermined structure is included in the two-dimensional structural formula of the predicted target compound, the arranging includes arranging the predicted target compound on the template at a position having the maximum degree of overlapping between the predetermined structure of the template and the similar structure.

**8.** A metabolic analysis apparatus, comprising:
an arrangement processing unit (41) configured to arrange a predetermined structure of a predicted target compound which is in a structural formula so as to be in an identical position with a predetermined structure of a template produced by superimposing structural formulae of a plurality of compounds capable of forming a complex with a predetermined enzyme; and
a determination unit (42) configured to determine, if the structural formula of the predicted target compound arranged on the template by the arrangement processing unit (41) overlaps with a metabolic target site of the predetermined enzyme in the template, that the predicted target compound is a metabolizable compound which can be metabolized by the predetermined enzyme, **characterised in that**
the structural formulae of the predicted target compound and of said plurality of compounds used to produce said template are two-dimensional structural formulae;
said arrangement processing unit (41) and said determination unit (42) arranged to operate on each of a plurality of arrangement states of the predicted target compound with respect to the template; and further **characterised by**
a score calculation unit (43) configured to calculate a score indicating a likelihood that the metabolic target site of the metabolizable compound determined by the determination unit (42) in each arrangement state will be metabolized by the predetermined enzyme based on a level of contribution of each atom included in the template during a metabolic reaction of the predetermined enzyme;
wherein said determination unit (42) is arranged to prioritize the arrangement states in order of higher score calculated by the score calculation unit (43), and to predict metabolites of the metabolizable compound only for the metabolic target site in arrangement states having a difference from the highest score within a predetermined range.

**9.** The metabolic analysis apparatus according to claim 9, wherein the determination unit (42) is arranged to determine that, among predicted target compounds determined as being the metabolizable compounds, predicted target compounds determined as being unable to access a metabolic active center site of the predetermined enzyme and predicted target compounds having the same charge as that of the metabolic active center site of the predetermined enzyme are compounds which cannot be metabolized by the predetermined enzyme.

**10.** A metabolic analysis method, comprising:
arranging a predetermined structure of a predicted target compound which is in a structural formula so as to be in an identical position with a predetermined structure of a template that is read from a template storage unit (31) and produced by superimposing structural formulae of a plurality of compounds capable of forming a complex with a predetermined enzyme;
determining, if the structural formula of the predicted target compound arranged on the template by the arranging overlaps with a metabolic target site of the predetermined enzyme in the template, that the predicted target compound is a metabolizable compound which can be metabolized by the predetermined enzyme, and storing the metabolizable compound thus determined in a metabolizable compound storage unit (32); **characterised by**:
the structural formulae of the predicted target compound and of said plurality of compounds used to produce said template being two-dimensional structural formulae;
performing the arranging and determining for each of a plurality of arrangement states of the predicted target compound with respect to the template;
calculating a score indicating a likelihood that the metabolic target site of the metabolizable compound determined by the determining in each arrangement state will be metabolized by the predetermined enzyme based on a level of contribution of each atom included in the template during a metabolic reaction of the predetermined enzyme; and by
prioritizing the arrangement states in order of higher score calculated by the calculating, and predicting metabolites of the metabolizable compound only for the metabolic target site in arrangement states having a difference from the highest score within a predetermined range.

**11.** The metabolic analysis method according to claim 10, wherein the determining includes determining that, among predicted target compounds determined as being the metabolizable compounds, predicted target compounds determined as being unable to access a metabolic active center site of the predetermined enzyme and predicted target compounds having the same charge as that of the metabolic active center site of the predetermined enzyme are compounds which cannot be metabolized by the predetermined enzyme.
